# EUROPEAN PATENT APPLICATION

(11) **EP 3 178 469 A2**
(43) Date of publication of application: **14.06.2017**
(21) Application number: 15772009.5
(22) Date of filing: 05.08.2015
(51) Int. Cl.: A61K 9/00, A61K 45/06, A61K 47/26, A61K 47/32, A61K 47/38, A61K 47/42, A61K 9/12, A61K 9/70, A61K 31/375, A61K 31/415, A61K 31/4174, A61K 31/7056, A61K 31/722, A61K 31/733, A61K 35/747

(54) **VAGINAL COMPOSITION FOR THE TREATMENT OF UROGENITAL INFECTIONS**

(30) Priority: 05.08.2014 PT 10783414
(71) Applicant: HPRD-Health Products Research and Development LDA, Covilhã (PT)
(72) Inventor: PALMEIRA DE OLIVEIRA, Ana Cristina, 6200-590 Peraboa (PT); PALMEIRA DE OLIVEIRA, Rita Manuela, 6200 Covilhã (PT)
(74) Representative: Patentree, Lda.
(86) International application number: PCT/IB2015/055948
(87) International publication number: WO 2016/020861

(57) **Abstract**

The present solution refers to compositions for films or solid foams, and method of preparation thereof, for prevention and treatment of urogenital infections, particularly vaginal infections.

A composition of aqueous base is described for a film/solid foam for use in the treatment of urogenital infections comprising:
0.4-25 % (w/w) of a plasticizer,
0.4-20 % (w/w) of at least a mucoadhesive polymer,
At least a probiotic and/or prebiotic,
At least an active substance in pharmacologically effective amounts.

The compositions/films/solid foams described in the present disclosure can be used in the medical field and pharmaceutical industry, particularly for the local treatment of urogenital diseases, particularly vaginal infections.

## Description

### Technical field

The present solution refers to compositions for films or solid foams (sponges), and methods of preparation thereof for prevention and treatment of urogenital infections, particularly vaginal infections.

The use of the compositions/films/solid foams described in the present disclosure in the medical field and pharmaceutical industry, particularly for the local treatment of urogenital diseases, particularly vaginal infections.

### Background

Urogenital infections (vaginal bacteriosis, urinary tract infection and vaginitis) affect more than one billion women per year, all over the world, representing the main reason for women to seek medical consultation. The three main causes of vaginal problems are trichomoniasis, vulvovaginal candidosis and vaginal bacteriosis.

Vaginal bacteriosis (VB) affects 10-15% of reproductive aged women and it is estimated that 50% of women are asymptomatic. VB is a complex condition, occurring due to the change in the normal vaginal flora dominated by *Lactobacillus* spp. for a polimicrobial community of other aerobes and anaerobes, namely *Prevotella* spp., *Mobilluncus* spp., *Gardenerella vaginalis* and *Mycoplasma hominis.* The decrease in the number of *Lactobacillus* spp., microorganisms responsible for the production of lactic acid that confers acidic pH (3.5-4.5) to the vaginal milieu, explains, in this affection, the usually high pH. A VB situation is sintomatologically characterized by excessive vaginal discharge produced, pH increase and unpleasant odor, being therefore associated with high discomfort. VB is regularly associated with vaginal hygiene and sexual behavior, although it is not considered to be a sexually transmitted infection. It is common during pregnancy and can cause negative outcomes such as premature rupture of membranes and low birth weight infants. It is estimated that more than 30% of women experience a recurrence within 3 months after treatment. The fact that this infection is related to disturbances of the normal flora has raised the interest in using protective microorganisms (probiotics) to recolonize the vagina in the treatment and prevention of the recurrence.

The use of vaginal probiotics (especially those belonging to the genus *Lactobacillus*) has been also studied in other clinical and physiological conditions such as the recurrence of urinary infections and menopause where the imbalance of the vaginal commensal flora has also been shown. This therapeutic and preventive approach is strongly associated with the protective local effect by exploration of the several mechanisms of action associated to probiotic microorganisms.

Among these, there are the direct competition in relation to the nutrients and to adhesion sites of epithelial cells, the production of substances such as hydrogen peroxide and bacteriocins that are highly active against pathogen microorganisms as well as organic acids (lactic and formic) that maintain the pH which is hostile for the development of pathogenic bacteria. These protective bacteria further exhibit the ability to modulate the local immune system. The combination of these mechanisms of action confers to probiotics a wide spectrum of activity when compared with conventional therapies.

According to the Portuguese Pharmacopoeia (FP 9.0) and the European Pharmacopoeia 8.0 the vaginal preparations include liquid preparations, semi-solid or solid to be administered vaginally, mainly for local effects. They contain one or more active substances in a suitable excipient. Several categories of vaginal preparations are identified: pessaries, vaginal tablets, vaginal capsules, vaginal solutions, emulsions and suspensions; tablets for vaginal solutions or suspension; vaginal semi-solid preparations, solid foams and medicated vaginal tampons.

In practice, the commonest pharmaceutical forms to be vaginally administered are pessaries, vaginal tablets and ointments. They are used to deliver adstringent drugs, anti-infectious (antibacterial, antiprotozoan, antifungal and antiviral), keratoplastics, skin healers, spermicides, prostaglandins and steroids. However, the traditional commercial preparations, such as creams, solid foams, gels, irrigation solutions and tablets, have been associated with reduced retention, due to the self-cleaning action of the vagina that is associated with discomfort and leakage sensation; they are uncomfortable during application; they often require multiple daily administrations in order to achieve the desired therapeutic outcome and they do not promote the evenly drug distribution. These characteristics often lead to decreased acceptability of vaginal pharmaceutical forms by women which, then, reduces the efficacy of the treatment which, in turn, is also affected by the difficulty of uniform spreading of the drug on the vagina. In a recent study promoted by the authors of this patent, that involved 2500 women with the goal of evaluating their previous experiences, preferences and perspectives regarding the use of vaginal products, it was shown that although the vaginal route is considered by women more effective than the oral route, its use can be limited essentially by comfort issues.

Technological evolutions together with the need to respond to therapeutic needs while increasing acceptability of vaginal pharmaceutical forms have promoted the development of new types of devices and pharmaceutical forms such as the vaginal rings and the vaginal films. Furthermore, new systems have been developed to increase the retention time in the application site in order guarantee drug concentration after one single administration. These systems are based on polymers with bioadhesive properties.

The retention, leakage, absorption kinetics and bioactivity of vaginal formulations rely on their interaction with the vaginal fluids.

The vaginal films pharmaceutical form consists of a thin and small sheet of polymeric hydrophilic substances that disperse or dissolve in contact with vaginal fluids to release the active substance. Vaginal films are pharmaceutical forms that are convenient, discrete, they do not require the use of an applicator to be administered and, as they disperse in vaginal fluids, they have low risk of increasing fluids volume and leak out, therefore improving comfort of use and efficacy after administration. Other advantages of vaginal films include their portability and low cost per unit. Since they are solid pharmaceutical forms, films can vehicle drugs that are susceptible to hydrolytic degradation, guaranteeing their stability. They can be formulated for immediate or sustained drug release.

Although scarcely explored from the commercial point of view, vaginal films are already in use as vaginal pharmaceutical forms. One example is Vaginal Contraceptive Film (VCF), which is currently marketed in United States of America and contains the spermicidal agent Nonoxynol-9. The development and study of vaginal films has been largely focused on their potential to reduce the risk of sexually transmitted infections through delivery of drugs internationally known as microbicides and, less frequently, on their use as vehicles for antifungals and antibacterials, in the context of the commonest vaginal infections.

In general, films are obtained by solvent casting, by drying the compositions in ovens to obtain very thin structures.

The aspects previously mentioned show the technical problem which is addressed by the present solution.

### General Description

Female urogenital infections are characterized by being highly prevalent among the female population and the currently available vaginal pharmaceutical forms reveal several disadvantages, namely:
- Inconvenient under the users' perspective, therefore limiting compliance and
- Efficacy limitations in some clinical conditions.

The present disclosure relates to vaginal compositions of aqueous base, in the final form of film or sponge/solid foam of variable thickness depending on the composition and method of preparation, particularly one vaginal film/solid foam, and method of production thereof, for treatment and prevention of urogenital infections, particularly vaginal infections.

The present disclosure, as a whole, describes compositions for films or solid foams for the treatment of urogenital diseases, with viable probiotics that promote not only the prevention/treatment of symptoms and the development of commensal flora. This solution thus allows the relief of symptoms and the prevention of repeated visits to the gynaecologist, reducing the risk of irritation of the delicate skin - and adverse effect that is common in similar compositions.

The compositions containing probiotics for the treatment of urogenital diseases described in the present disclosure can include polymers with bioadhesive capacity (chitosan, hydroxipropilmethylcellulose, hydroxiethylcellulose, hydroximethylcellulose, sodium carboximethylcellulose) and macromolecules (gelatin) selected by their high biocompatibility. These compositions allow for, where there is dispersion by contact with vaginal fluids (films), the obtention of *in loco* gels with increased vaginal retention on administration site, being therefore associated with higher therapeutic efficacy and acceptability by users.

To date, no other probiotic formulation in the form of vaginal film or solid foam is known. This pharmaceutical form is unique since, in practice, it combines two technologies in one formulation: solid form and gel (in which it is converted after application). Following hydration, the gel covers the surface of the vaginal mucosa. On the other side, this pharmaceutical form is comfortable to apply, has low risk of increasing the vaginal fluids and therefore presents high application comfort, reduced increase of vaginal fluids with low leakage potential and is also able to deliver chemical or biological entities which easily undergo degradation in contact with water.

Throughout the present document, a vaginal film is understood as a pharmaceutical form with low thickness which allows for the dispersion or dissolution in contact with vaginal fluids to release the vehicle substances or simply form a gel exerting a therapeutic or preventive action.

Throughout the present document, probiotics are understood as "live microorganisms that, when administered in adequate amounts, confer benefit to the health of the host" (FAO/WHO, 2001).

Throughout the present document, prebiotics are understood as non-digestible nutritional ingredients that beneficially affect the host by selectively stimulating the growth and activity of one or more beneficial bacteria, improving the health of the host".

Throughout the present document, pharmabiotic is understood as a product formulation that contains along with live beneficial microorganisms, dead cells, cellular metabolites or other molecules such as hormones, which contribute to improve the health of the host.

Throughout the present document, active substance is understood as a substance which will exert a pharmacological effect, i.e. a therapeutic agent or drug.

The film compositions included in the present disclosure differ from the previously published and marketed compositions in their formula and activity and include the administration of live microorganisms with probiotic effect or components that stimulate the proliferation of the protective flora promoting recolonization of vaginal flora. This application assumes additional innovation since, to date, no probiotic films/solid foams had been described for vaginal application. Therefore, the compositions in this disclosure differ from the previously disclosed in their composition and technological characteristics. The present disclosure is different regarding:
- recommended dimensions (designed to cover the surface of the vagina)
- technology used in the preparation thereof;
- combination of excipients (with protective properties regarding the formulation and bioactivity);
- application to different vaginal diseases and behavior in contact with the vaginal fluids, i.e. *in loco.*

In an embodiment, the use of probiotics as a prevention strategy for infection recurrence and vaginal flora recolonization in particular conditions has been considered only with the administration of conventional pharmaceutical forms, particularly capsules, tablets or pessaries. In this context, the present solution represents the development of novel pharmaceutical forms or formulations of vaginal application, to effectively treat or prevent infections, designed to fulfill users' preferences. This presentation presents high efficacy potential since it covers the surface of the vagina after dispersion, and high compliance potential since it does not promote the increase of the vaginal fluids (which is associated with leakage) and presents bioadhesive potential.

In an embodiment, chitosan has been largely studied as an excipient with antibacterial and antifungal potential and its interest as excipient for the treatment of vaginal infections has been studied by the authors of this patent. Its inclusion in film compositions of the present disclosure, at dispersion promoted by the present vaginal fluids, boosts its antimicrobial activity, to promote the treatment of the infection with high level and comfort. The additional use of lactic acid (the vaginal physiological acid) included or not in a buffer solution compatible with the normal physiological environment, aims at correcting and help maintaining the normal vaginal pH to improve the efficacy of the treatment and prevent infections recurrence by promoting vaginal recolonization by the protective flora.

The present disclosure differs from the vaginal sheets, described in the background, for comprising a variable thickness depending on the method used for drying (varying from 0.1 mm to 1-2 mm, if dried in oven or lyophilized) and for rapidly dispersing in contact with vaginal fluids, releasing the vehicle substances, while the vaginal sheets are designed to be retained in the vaginal cavity for absorption and being able of being removed or naturally expelled.

Additionally the present disclosure differs from the products previously known in its composition and *in vitro* activity as shown, including probiotics, which had not been previously published.

An aspect of the present disclosure refers to a composition of aqueous base for a film or solid foam for use in the treatment of urogenital infections comprising:
0.4-25 % (w/w) of a plasticizer,
0.4-20 % (w/w) of at least a mucoadhesive polymer,
at least a probiotic and/or prebiotic,
at least an active substance in pharmacologically effective amounts.

The final appearance of the compositions depends mainly on the drying method used: solvent casting through drying by heat results in very thin and pliable compositions that exhibit film appearance while lyophilization of the same compositions results in opaque preparations whose polymeric structure remains unaltered (the thickness of the formulation is the same as the composition of aqueous base which served as basis). In both cases, the compositions are solid, soft, pliable, and are easy to apply without the use of an applicator and have the ability to disperse in contact with the vaginal fluids.

In an embodiment, the compositions containing probiotics for the treatment of urogenital diseases described in the present disclosure can comprise:
0.5-10% (w/w) of a plasticizer,
0.5-5% (w/w) of at least a mucoadhesive polymer;
0.25 - 10% (m/m) of at least a probiotic, preferably 1-2.5% (w/w)
0.25-15 % (w/w) of an active substance, preferably 0.5-3% (w/w).

In an embodiment, the compositions containing probiotics for the treatment of urogenital diseases described in the present disclosure can comprise 10⁵ - 10¹² CFU/ml of a probiotic culture.

In an embodiment, the probiotics of the compositions for the treatment of urogenital diseases described in the present disclosure can be, among others, lactic acid bacteria, particularly Lactobacillus spp. The Lactobacillus species can be preferably selected from a list consisting of: Lactobacillus iners, Lactobacillus crispatus, Lactobacillus acidophilus, Lactobacillus delbruekii, Lactobacillus jensenii or mixtures thereof, among others.

In an embodiment, the active substance of the compositions containing probiotics for the treatment of urogenital diseases described in the present disclosure can be selected from a list consisting of: metronidazole, clyndamicin, clotrimazole, chitosan, oligochitosans or combinations thereof, among others.

In an embodiment, the compositions containing probiotics for the treatment of urogenital diseases described in the present disclosure can also comprise a prebiotic agent, surfactant, dye, vitamin, antioxidant, hormones and/or cryoprotectant agent, among other additives.

In an embodiment, the prebiotics of the compositions containing probiotics for the treatment of urogenital diseases described in the present disclosure, can be selected from a list consisting of: inulin, lactose, ascorbic acid, skim milk, fructooligoasaccharides, glucose, lactic acid, sodium lactate, or combinations thereof, among others.

In an embodiment, the cryoprotectant agent from the compositions containing probiotics for the treatment of urogenital diseases described in the present disclosure can be selected from a list consisting of: trehalose, glycerol, lactose, glucose or combinations thereof, among others.

In an embodiment, the mucoadhesive polymer of the compositions containing probiotics for the treatment of urogenital diseases described in the present disclosure can be selected from a list consisting of: gelatin, polyvinyl alcohol, carbomers, chitosan, polycarbophil, cellulose derivatives or combinations thereof, among others. In these compositions, chitosan can be avoided to prevent the premature death of probiotic microorganisms.

In an embodiment, the cellulose derivatives of the compositions containing probiotics for the treatment of urogenital diseases described in the present disclosure can be selected from hydroxypropylmethylcellulose, hydroxyethylcellulose, sodium carboxymethylcellulose, hydroxymethylcellulose, ethylcellulose, thyme extracts, Helichrysum spp. extracts, Echinacea spp. extracts, Satureja spp. extracts, clove extracts and combinations thereof.

In an embodiment, the used chitosan can have molecular weight between 10-150 kDa or 310-350 kDa.

In an embodiment, the compositions containing probiotics for the treatment of urogenital diseases described in the present disclosure, can contain: 0.4-5% (w/w) of cellulose derivatives and 0.1-2% (w/w) of lactic acid, or sodium lactate, or mixtures thereof.

In an embodiment, the celullose derivatives of the compositions containing probiotics for the treatment of urogenital diseases described in the present disclosure can be a poliol, preferably selected from the list consisting of: propane-1,2 diol, propane-1,2,3 triol, glycerol, polyethilenoglycol, or combinations thereof.

Another aspect of the present disclosure relates to the use of compositions containing probiotics for the treatment of urogenital diseases described in the present disclosure in the prevention and/or treatment of vaginal infections, particularly those caused by Gardnerella vaginalis, Prevotella spp., Mobilluncus spp., Mycoplasma hominis, Candida spp., or Trichomonas vaginalis.

Another aspect of the present disclosure relates to a film obtainable through dehydration of the composition containing probiotics and/or prebiotics for the treatment of urogenital diseases described in the present disclosure through solvent casting, particularly by heat (example: 45ºC in oven during 18h-24h) a very thin preparation being obtained, with a transparent or only slightly opaque appearance, typical from films, very soft and pliable.

In an embodiment, the film for the treatment of urogenital diseases described in the present disclosure can present a thickness between 0.09 - 3 mm, preferably 0.25 - 2 mm.

In an embodiment the film for the treatment of urogenital diseases described in the present disclosure can present a substantially rectangular shape with length between 5-10 cm and 2-4 cm side.

Another aspect of the present disclosure relates to a polymeric preparation, opaque and white as a solid foam and with organoleptic resemblances to a sponge but with a compact appearance and with ability to disperse, obtainable by lyophilization of the compositions containing probiotics for the treatment of urogenital diseases described in the present disclosure. In these cases the preparation is frozen (at -20ºC or -80ºC), the composition of aqueous base is poured in adequate molds and the water removal is made through the use of a freeze-drier (example conditions: minimum temperature achieved -110ºC, pressure 0.019hPa; duration: 24h). The obtained preparation retains the initial thickness and acquires a white appearance, opaque, with an ability to disperse superior to the films, when in contact with vaginal fluids.

In an embodiment the solid foam for the treatment of urogenital diseases described in the present disclosure can present 2mm thickness (1-3mm), obtained by the proportional use of 2.72g of composition of aqueous base in circular molds of 3.5 cm diameter.

In an embodiment the vaginal film compositions now disclosed can contain chitosan and exhibit antibacterial and antifungal activity to be used for the treatment of infections.

In an embodiment, the obtained compositions correspond to vaginal films, in particular transparent or slightly opaque films and very thin (approximately 0.1 mm thickness) or opaque and 1-3 mm approximate thickness. They allow the delivery of probiotics, preferably *Lactobacillus* spp., to recolonize the vagina, as a strategy to rebalance the vaginal flora which contributed to the prevention of infections. In these compositions, chitosan can be avoided to prevent the premature death of microorganisms.

### Brief description of the drawings

The following figures provide preferred embodiments for illustrating the description and should not be seen as limiting the scope of the disclosure.

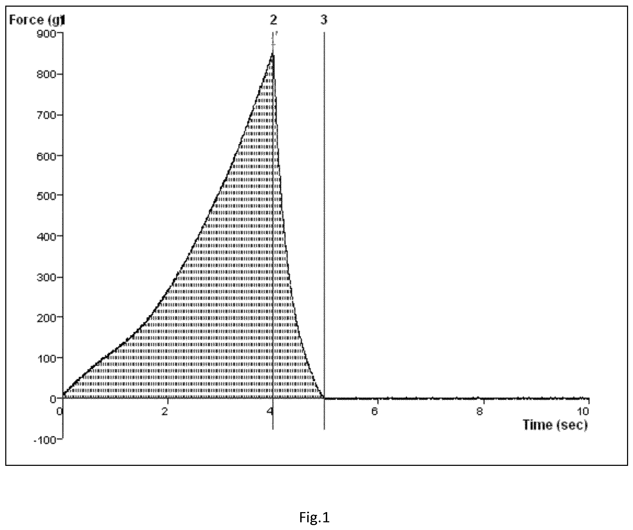

**Figure 1****:** Graphical representation of resilience measurements: wherein (1) indicates the area 1 and (2) indicates the area 2. Resilience value was calculated by the ratio between area 2 and area 1, in percentage.

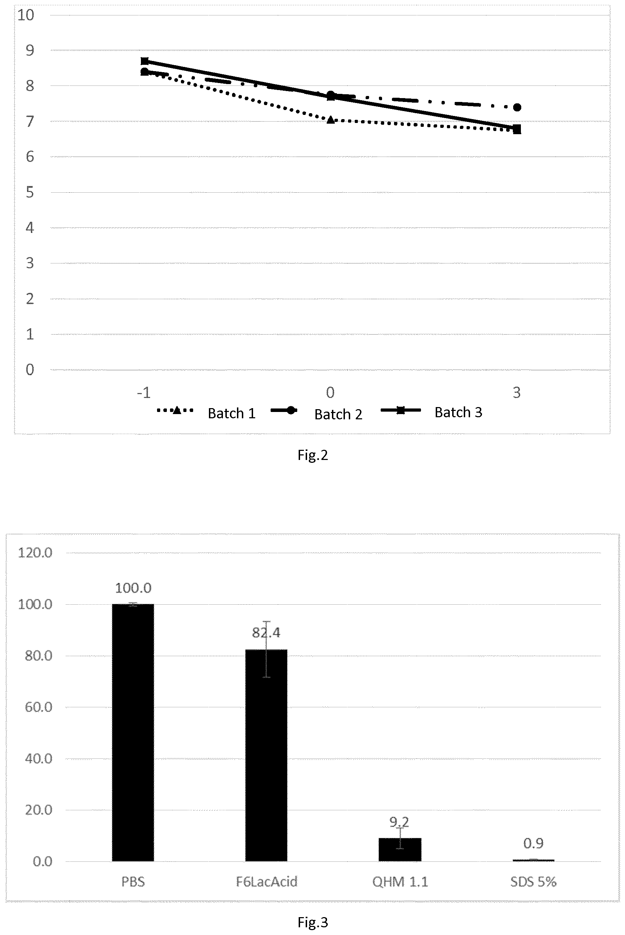

**Figure 2****:** Graphical representation of *L. acidophillus* viability, in a film formulation (presented as example) (F6) immediately after drying by freeze-drying (time 0) and after, at least, 3 months storing in refrigerated conditions (2-8 °C) (time 1). Time -1 corresponds to initial counting of microorganisms incorporated in the formulation (counts per film).
**Figure 3****:** Graphical representation of cytotoxicity of exemplifying film formulations containing *L. acidophillus* (F6) and containing chitosan (F11 = QHM 1.1) aimed at the prevention and treatment of vaginal infections, respectively. Results were obtained by the MTT test using PBS (Phosphate Buffer Saline) as negative control (100% viability) and SDS (Sodium disulfate 5 %) as positive control. The tissue used corresponds to a commercial model of acute vaginal irritation (Reconstructed human vaginal epithelium HVE - Skin Ethic^{®}) with 12h incubation of the formulations (17 mg of film were added to 17 µL of PBS to promote dispersion).

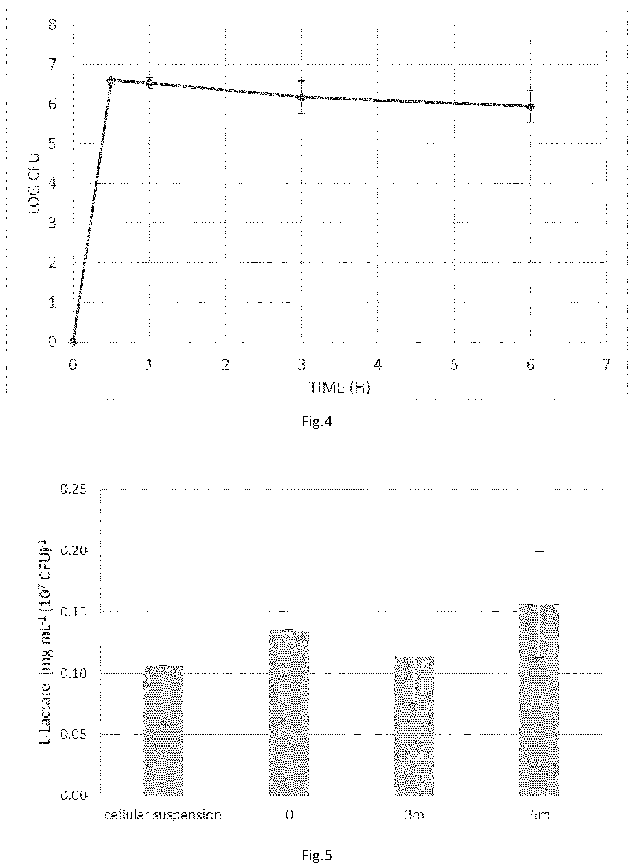

**Figure 4****:** Graphical representation representing rapid release of probiotic microorganisms from a lyophilized formulation (F6) after dispersion in a small volume (5mL) of saline solution.
**Figure 5****:** Graphical representation and representative of the maintenance capacity of L-lactate by *L.acidophilus* after formulation and storage of films, in refrigerated conditions. cellular suspension = Control (cellular suspension), after formulation = t0, after storage of films (2-8ºC) for 3 months (3m) and 6 months (6m). The results are expressed as mg/mL of L-Lactate produced by 10⁷ CFU, after 20h of incubation of the dispersions obtained from the films.

### Detailed Description

The present disclosure relates to compositions containing probiotics and/or prebiotics for use in the treatment and prevention of vaginal infections, in the form of film or solid foams. The technology involved in the preparation of the compositions herein described allows for the obtention of vaginal film/solid foam formulations with compositions for the treatment or prevention of these infections, including the delivery of live microorganisms with probiotic activity. The incorporation of probiotic microorganisms in vaginal films of polymeric and natural base allows for the maintenance of their viability and probiotic properties and their rapid dispersion after application without generating additional fluids which are associated to leakage and discomfort for the users.

The present disclosure relates to vaginal compositions of aqueous base, corresponding to the final form of a film or sponge/solid foam - with variable thickness depending on the composition and method of preparation, particularly a vaginal film, and method of production, for the treatment and prevention of urogenital infections, particularly vaginal infections.

One aspect of the present disclosure refers to a composition of aqueous base for a film or sponge/solid foam for use in the treatment of urogenital infections comprising:
0.4-25 % (w/w) of a plasticizer,
0.4-20 % (w/w) of at least a mucoadhesive polymer,
at least a probiotic and/or prebiotic,
at least an active substance in pharmacologically effective amounts.

The use of the compositions/films/solid foams described in the present disclosure in the medical field and pharmaceutical industry, particularly for the local treatment of urogenital diseases, particularly vaginal infections.

In an embodiment the developed vaginal films are thin, very pliable, easily placed at the top of a finger and inserted in the vagina. Depending on the method of preparation these films can be transparent, with thickness inferior to 1mm (if dried in oven) or white opaque preparations (with similar appearance to solid foam/ styrofoam) soft and malleable in consistency, being easily pliable to be applied as described and rapidly dispersing when in contact with the vaginal fluids.

In an embodiment, the shape, size and mode of application of the vaginal films leads to a an adjusted covering of the vaginal cavity since, by using adequate molds or by dimension adjustment (cutting) after drying, it is possible to obtain final products that fit the vaginal cavity, for example, rectangles with 7x2.4 cm (length versus side).

In an embodiment, the films containing probiotics described in the present disclosure allow a rapid dispersion in vaginal fluids originating a gel which, due to its composition in bioadhesive polymers, increases the retention time of the formulation in the application site and avoids the discomfort related to product leakage. In an embodiment, the compositions developed without chitosan allow the better delivery of live microorganisms such as *Lactobacillus* spp. in a lyophilized final form. The formulations/compositions containing chitosan present intrinsic antimicrobial activity and can, additionally, deliver chemical drugs for the treatment or prevention of different vaginal affections. The drugs/substances that can be delivered by the vaginal film are, for instance, metronidazole, clindamycin, extracts obtained from plants such as thyme, *Helichrysum spp., Echinacea spp., Satureja* spp., among others or combinations thereof.

In an embodiment, the compositions/films containing probiotics for the treatment of urogenital diseases described in the present disclosure can comprise the mixture of the following excipients, particularly:
polymers and macromolecules with mucoadhesive potential, such as gelatin which is considered safe for vaginal use;
chitosans with molecular weight in the range 10-350 KDa, as well as oligochitosans which molecular weight is inferior to 10 KDa;
polyvinyl alcohol;
polyols (plasticizers/humectants) preferably glycerin since it has been shown to be safer for vaginal use, but it is also possible to use propyleneglycol and/or polyethyleneglycols probiotic microorganisms such as Lactobacillus spp.
prebiotics and/or cryoprotectants such as ascorbic acid, skim milk, trehalose, lactose, glucose, fructooligosaccharides (FOS), inulin, starch, lactic acid;
sodium lactate; bacteriocins;
water.

In an embodiment, the compositions containing probiotics described in the present disclosure can be obtained according to the following steps:
1. Weighting the components to incorporate in each formulation;
2. Turning on the thermostated water bath or orbital shaker at 45-50ºC;
3. Dispersing the polymers (chitosan, cellulose derivatives) in the aqueous base (which can or cannot contain lactic acid and sodium lactate or other acid such as acetic acid) by mechanical stirring (helix stirrer);
4. Adding the gelatin and dissolving in the orbital shaker under shaking;
5. Adding the plasticizer and shaking;
6. Pouring the formed gel to plastic Petri dishes or molds (previously patterned regarding the amount of gel to deliver: for example, 18 g for films in 90 mm diameter Petri dishes or 2.72 g for smaller Petri dishes (3.5 cm diameter); For formulations containing probiotics, the mixture with microorganisms is performed at room temperature;
8. Freezing at -80 °C *over night* (for obtaining the thicker films - solid foams);
9. Lyophilizing by freeze-drying, for 24h (the temperature reached is -118ºC).
   or
10. Drying in oven at 45ºC, for 18-24h (films).

In an embodiment, the compositions containing probiotics described in the present disclosure do not contain gelatin, do not need heating during preparation. Dissolution is achieved by a helix stirrer.

In an embodiment, the compositions containing probiotics described in the present disclosure can be prepared in molds or 90 mm diameter Petri dishes and then be cut to the adequate dimension for vaginal application (the suggested dimension is 7 × 2.4 cm to cover the whole vaginal cavity). Alternatively, customized molds with the final film dimensions can be used.

In an embodiment, the packaging of the compositions/films/solid foams containing probiotics described in the present disclosure, must assure the contact with low levels of humidity throughout storage.

In an embodiment, the preparation of the compositions containing probiotics described in the present disclosure, particularly *Lactobacillus* spp. requires the previous preparation of the microorganisms in cellular suspension and mixture with prebiotic and cryoprotectant excipients, so as to be included in the polymeric base gel before the freezing and drying steps. The prebiotic excipients can be selected from the following list: lactose, skim milk, ascorbic acid, lactic acid, sodium lactate, inulin, fructooligosaccharides, glucose, trehalose, among others and combinations thereof.

In an embodiment, the organoleptic characterization of the formulations can be performed according to the following parameters: appearance, color, opacity and homogeneity; odor; softness and malleability. Malleability includes the ease of the composition to be winded and its posterior ability to return to its original shape (which can be important to guarantee that it covers the walls of the vaginal cavity) and the ease of folding the film/solid foam for its application.

In an embodiment, the vaginal films obtained through dehydration/drying of the compositions described in the present disclosure are soft and easily handled, are transparent when dried in oven or white and opaque, with polymeric appearance, if lyophilized through *freeze-drying.*

In an embodiment, the vaginal film formulations described in the present disclosure can be evaluated concerning the following technological parameters: texturometric parameters, thickness, pH of the dispersion of the formulation in normal saline and in a vaginal fluid simulant. The texturometric parameters evaluate the hardness and resilience of vaginal films, as it can be seen in figure 1. The hardness of the vaginal films was determined by the maximum force (Fmáx) exerted by the texturometer (Ta XT Plus) probe (2 mm diameter, needle shape) over a sample, under the experimental conditions. That means that the higher the hardness of the formulations, the higher the Fmáx. The resilience of all formulations was determined using a 2 mm diameter probe with flat termination which pushes the sample through a platform with central aperture (pre-test speed: 1 mm/sec; test speed: 0.5 mm/sec). After the initial contact the probe moves further 2 mm and then returns to its initial position (post-test speed 0.5 mm/sec). The film recovers from deforming as the probe is raised, and the film retraction exerts an upward force on the probe, causing a gradual decline in force of the probe. Resilience of the formulations was determined, by calculation, as the ratio of the graphical areas obtained during the ascending and descending period of the probe.

In an embodiment, for vaginal films it was observed that formulations with higher hardness (identified even in the organoleptic evaluation) corresponded to the ones with lower resilience and in some cases undergone rupture during the test. It is intended that these formulations are malleable enough to be inserted in the vagina without breaking upon manipulation.

In an embodiment, the thickness of vaginal films formulations was measured by using regular rulers and a digital micrometer (for films thinner than 1 mm, obtained by drying in oven). Formulations obtained by *freeze-drying* present thickness ranging from 1 to 2 mm, are very malleable and have a little compact structure. Vaginal film formulations dried in oven present a thickness varying from 90 µm (for F7 sample which base composition is gelatin 2.5%, glycerin 0.8% and water/lactate buffer 96.7%) and 200-250 µm for F6 (HPMC 1%, gelatin 2.5%, glycerin 2.5%, water/lactate buffer 94%) and QHM 1.1 (HPMC 2.5%, chitosan 1%, glycerin 2.5% or glycerin 0.8 and water/lactate buffer quantum satis ad 100).

In an embodiment, the pH of the dispersion of the formulation was studied in normal saline and in a vaginal fluid simulant (pH 4.2 adjusted with HCl 1M) with the following composition: 1.76 g NaCl; 0.70 g KOH; 0.111 g Ca(OH)₂; 0.009 g bovine serum albumin; 1.00 g lactic acid; 0.50 g acetic acid; 0.08 g glycerol; 0.2 g urea; 5.0 glucose; water quantum satis ad 500 mL.

In an embodiment, the evaluation of pH and buffer capacity of vaginal formulations assumes particular relevance since bacterial vaginal infections are characterized by causing changes in the vaginal pH. It is, therefore, important to know the final pH of the formulations and their ability to maintain it (buffering capacity) so as to adjust it, which is unfavorable to pathogenic microbial growth while favoring the growth of normal vaginal flora.

In an embodiment, the study of the buffering capacity was based on the methodology described for vaginal gels and was performed as follows:
- The formulation was dissolved, at 37ºC, in the 1:20 proportion of film weight: dissolvent;
- The dissolution was performed in NaCl 0,9 %, VFS pH=4 e VFS pH=5;
- The initial pH of the dissolution was measured;
- 20 µL of NaOH 1N were added to the solution, and the pH obtained was measured;
- The previous procedure was repeated twice or until pH be equal or higher than 7;
- Control assays have been performed for NaCl 0.9 %, VFS pH=4 e VFS pH=5, without adding formulations.

In an embodiment, for the films, since they completely disperse in contact with the vaginal fluids, pH was also measured after dispersion of a formulation in 3 mL NaCl 0.9 % and VFS pH=4.2.

**Table I - examples of vaginal film compositions for prevention through inclusion of probiotics (without chitosan as active substance) and their technological comparison with the base formulation pH of its dispersion in normal saline (NS) and vaginal fluid simulant (VFS)**

| | Composition (g) + water q.s. ad 100g) | Dimension (cm) | Mass (g) | Hardness (Fmáx) | Resilience (%) | Contact SFV (%) pH=4 | Contact SFV (%) pH=5 | pH (in NaCl) |
|---|---|---|---|---|---|---|---|---|
| QHM 1.1 (film) | 2.5% HPMC; 1% chitosan; 2.5% Glycerine; lactic acid 2% | 7x2.4 | 0.420 | | | | | 4.23 |
| F6 | 1% HPMC; 2.5% gelatin; 2.5% glycerin. | 7x2.4 or the area of a small petri dish | 0.298 | | | | | 5.1 |
| F7 | 2.5% gelatin; 2.5% glycerin | | 0.140 | | | | | 5.1 |

**Table II - represents the initial pH of vaginal sheets and films in tests NaCl 0.9 %, VFS pH=4 and VFS pH=5**

| | Control | QHM 1.1 | F6 | F7 |
|---|---|---|---|---|
| NaCl 0.9% | 8.40 | 4.23 | 5.1 | 5.1 |
| VFS pH=4 | 4.02 | 3.97 | 3.8 | 4.1 |
| VFS pH=5 | 5.38 | --- | -- | -- |

The table shows the lyophilized composition F6/G6 and its technological characteristics. The dispersion of the film in vaginal fluid simulant has pH 4.5 +/-1 showing that this preparation does not change the normal pH of vaginal fluids. In normal saline the pH of the dispersion is 5.1 +/1.

Formulation QHM 1.1 (G11) allows for the correction of the pH and maintain it in the physiological range due to its high buffering capacity.

**Table III - represents the anti-Gardnerella spp. activity of films containing chitosan (QHM 1.1: 2.5 % HPMC; 1 % medium molecular weight chitosan; 2.5 % glycerin; lactic acid 2 % (v/v) q.s. ad 100; and QHM 0.8: 2.5 % HPMC; 1 % medium molecular weight chitosan; 0.8 % glycerin; lactic acid 2 % (v/v) q.s. ad 100. Films (cut to 7×2.4 cm dimension) were dispersed in 3 mL of water or vaginal fluid simulant (VFS) and tested by the micromethod, in clinical strains, in two serial concentrations.**

| | *G. vaginalis* G71 | | *G. vaginalis* G19 | |
|---|---|---|---|---|
| | 1:2 | 1:4 | 1:2 | 1:4 |
| QHM 1.1 in 3 mL H2O | - | - | - | - |
| QHM 0.8 in 3 mL H2O | - | - | - | - |
| QHM 1.1 in 3 mL SFV pH 6 | - | - | - | - |
| QHM 0.8 in 3 mL SFV pH 6 | - | - | - | - |
| SFV pH 6 | + | + | + | + |
| Lactic acid | - | - | - | - |

It was observed in some cases that films containing chitosan inhibit the growth of all tested strains due to its intrinsic activity and to the additional effect of lactic acid. No growth of the tested microorganisms was therefore observed in the wells where films previously dispersed in water or in VFS were added (even if this simulant has got altered pH).

**Table IV - represents the general characteristics of a vaginal film containing L. acidophilus, compared with a solution without prebiotics and/or probiotics (Blank)**

| | No of films tested | G6LB3 | G6 Blank | VCF^{®} |
|---|---|---|---|---|
| Weight (g) | 9 | 0.224±0.01 | 0.194±0.006 | 0.232 |
| Weight loss (%) | 6 | 93.22±0.15 | 93.50±0.20 | N.D. |
| Thickness (mm) | 6 | 1.636±0.6 | 1.917±0.3 | 0.275±0.01 |
| Osmolality (mOsm/Kg) | 3 | 306±0.6 | 296±5.8 | N.D. |
| Burst strength (g/mm) | 3 | 327±51 | 162±25 | 8173±589 |
| Distance at burst (mm) | 3 | 3.58±0.28 | 3.35±0.15 | 6.78±0.75 |
| Resilience (%) | 3 | 24.6±1.56 | 22.13±1.45 | 39.9±1.67 |
| Force at target distance (N) | 3 | 1.76±0.18 | 1.39±0.21 | 4.41±0.42 |

G6LB - vaginal film containing *L. acidophilus,* G6 Blank - vaginal film containing only the gel without prebiotics and probiotics, N.D. - not determined. Results represent the average value ± standard deviation (SD) of measurements on different tested films (n represents the number of tested films tested and not the total number of measurements).

The above table shows that the general technological characteristics of the base formulation are maintained by adding the probiotics and prebiotics, although a slight increase in total mass can be observed

In an embodiment, the pH of the dispersed formulations in VFS pH=4 and pH=5 is acidic comparing with NaCl 0.9%, demonstrating that the dispersions acquire pH similar to the fluids in which they are dispersed (Table II).

In an embodiment, vaginal films containing chitosan and containing lactic acid in their composition present lower pH following dispersion in NS and VFS. The use of a lactate buffer or lactic acid with adjusted pH redefines the pH of the dispersion within the physiological range.

In an embodiment, the viability of *L. acidophilus* in film formulations of gelatin base (F6) was studied after preparation by *freeze-drying* and throughout time. The microorganisms were cultured in MRS and used in the stationary phase. The culture was centrifuged and a cellular suspension was prepared in PBS up to 5x10⁷ UFC/ml (plate counts in MRS). For each film, 1 mL of suspension was centrifuged and resuspended in 1mL of optimized prebiotic mixture (containing skim milk, lactose and trehalose) and mixed with 2.72 g of gel formulation F6 and F7. After freezing at -80 °C, lyophilization was performed, particularly by *freeze-drying,* for 24h. The films obtained were dispersed in 5 mL of PBS e the microorganisms were counted by plate count immediately after lyophilization and up to 6 months after storage in the refrigerator (2-8 °C). The loss of viability during the drying process was about 0.9 log for formulation F6 and 3.2 for formulation F7. Formulation F6 presented reduced loss of viability of microorganisms after 6- months in the refrigerator, in the refrigerator. Figure 2 shows reduced loss of viability throughout 6 months storage, in refrigerated conditions, for 3 independent batches of formulation F6 (time 0 represents drying by *freeze-drying* and -1 represents counting of the initial suspension). It has also been demonstrated that the microorganisms formulated in these compositions maintain, even after storage, the biochemical characteristics that confer them probiotic characteristics, namely the production of lactic acid (Figure 5). This aspect is particularly relevant since the efficacy of the treatment depends on the maintenance of these properties. Previous studies have shown that formulation of microorganisms can limit the maintenance of these characteristics.

Figure 3 shows the low cytotoxicity of formulations containing probiotics when compared to control of PBS. For chitosan formulation toxicity is higher, as expected.

In an embodiment, the antibacterial activity of chitosan films was tested against two *Gardnerella vaginalis* clinical strains, this being the pathogen most frequently associated with vaginal bacteriosis. As an example, results from films of QHM 1.1 and QHM 0.8 dispersed in 3 mL of water or 3 mL of VFS with pH adjusted to 6 (the typical pH from vaginal bacteriosis) that were further diluted to half concentration, with culture media, in two sequential dilutions. The antibacterial activity was tested according to the CSLI M45 standard. Controls were performed with VFS and the lactic acid used for the preparation of formulations, as well as growth controls of strains and sterility of the culture medium used. All tests were performed in duplicate. Plates were observed concerning the presence or absence of bacterial growth after 48h of growth at 37ºC. Table 3 represents the results obtained indicating the marked activity of the film since no bacterial growth occurs for 1:4 dilution (second sequential dilution) of the film dispersion in 3mL of water and VFS. The cytotoxicity of film formulations containing *L. acidophilus* (F6) and containing chitosan (QHM 1.1) was tested in a commercial model of acute vaginal irritation (Reconstructed human vaginal epithelium HVE - Skin Ethic^{®}) corresponding to pluri-stratified epithelial tissue with similar histology to the normal human vaginal epithelium. The test was performed after incubation of the wells containing the tissue with maintenance medium (commercial), at 37 °C with 5 % CO₂ atmosphere, in accordance with the recommendations of the supplier. After the incubation period, 17 mg of film were placed over the tissue in each well and added of 17 µL of PBS to allow for dispersion. After the 12h incubation period, cellular viability was assessed by the MTT test (n=2 wells) and histological analysis was performed. The cellular viability results presented in Figure 3 show that, for the formulation containing lactobacilli, the viability is around 82% indicating its low toxicity and compatibility with the tissue which is required for a formulation developed for vaginal recolonization as a preventive strategy. For QHM 1.1 formulation, on the contrary, it was observed that the film presents high cytotoxicity for the tested concentration (9.2% compared with the negative control). Since this formulations was developed to treat an infection, being toxic to bacteria, it wouldn't be expected to totally innocuous for the human tissue. Biocompatibility of chitosan with human tissue has been described and the lactic acid used in this formulation was selected due to the natural occurrence in the vagina. On the other hand, the fact that this formulation is intended for short term treatments, in an altered vaginal environment due to the presence of pathogens, can influence the final effect of the formulation in the tissue of the host. Therefore, the real impact of these results on vaginal toxicity can only be studied through further toxicity testing and by comparing currently used treatments, in the same conditions.

Detailed compositions for vaginal films:
1 - Compositions containing gelatin (20-40 % w/w), propyleneglycol or glycerin (10-25 %) and water, in preferably proportional amounts of gelatin and humectant of 4:3 or 4:5, dried by freeze-drying.
2 - Compositions similar to those described in 1 containing a powder or mixture of powders up to 15% w/w.
3 - Compositions containing gelatin from 5%-20% w/w and propyleneglycol or glycerin ranging from 3 to 20%.
4 - Compositions containing gelatin in concentrations of 0-5 %, glycerin or propyleneglycol from 0.4-5 %, cellulose derivatives such as hydroxypropylmethylcellulose, hydroxyethylcellulose, sodium carboxymethylcellulose in concentrations of 0-5 %, lactic acid 0-2 %, sodium lactate 0-2 % and water (q.s. ad 100 g) dried in oven, at 45 °C up to 24h to obtain low thickness preparations called vaginal films.
5 - Compositions similar to those described in 4, frozen at -80 °C and lyophilized by freeze-drying to obtain formulations with the same composition but with a solid foam appearance.
6 - Compositions similar to those described in 4 and 5 containing a powder or mixture of powders up to 10% concentrations,
7 - Compositions similar to those described in 4 and 5 which include live microorganisms (probiotics such as those belonging to the genus Lactobacillus) mixed with prebiotic compounds such as inulin, lactose, ascorbic acid, skim milk, fructooligosaccharides, lactic acid, sodium lactate, trehalose, and glucose, and dried in oven at 45ºC up to 24h to obtain formulations with the appearance of films or solid foams, respectively.
8 - Compositions containing gelatin in concentrations 0-5%, glycerin or propyleneglycol 0.4-5%, chitosan with molecular weight in the range 310-150 kDa (commonly known as medium molecular weight chitosans) in concentrations ranging from 0-5%, celulose derivatives such as hydroxypropylmethylcellulose, hydroxyethylcellulose, sodium carboxymethylcellulose in concentrations ranging from 0-5 %, lactic acid 1-2 %, sodium lactate 0-2% and acetic acid 0-2% (q.s ad 100 g) dried in oven at 45 °C up to 24h to obtain low thickness preparations named vaginal films.
9 - Compositions similar to those described in 8 containing chitosans with molecular weight ranging from 10-150 kDa, commonly known as low molecular weight chitosans, or chitosans with molecular weight between 310-350 kDa, commonly known as high molecular weight chitosans.
10 - Compositions similar to those described in 8 and 9 containing a powder or mixture of powders up to 10% concentrations.
11 - Compositions similar to those described in 8, 9 and 10 dried by freeze-drying to obtain thicker preparations and with opaque appearance.

The vaginal films herein disclosed present several applications in the field of prevention and treatment of vaginal infections. As described, the use of formulations without chitosan allows the inclusion of live microorganisms with probiotic properties which promote the rebalance of the vaginal flora after infections or physiological unbalance. These formulations are compatible with probiotic microorganisms and exhibit low cytotoxicity.

Formulations with chitosan exhibit high potential to treat bacterial infections, namely vaginal bacteriosis, absorbing the excess of fluids in the vagina during treatment and rapidly dispersing therefore promoting homogeneous distribution in the vagina and increasing efficacy. Due to its composition, these formulations can also be applicable to fungal infections since chitosan exhibits antifungal activity.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples and drawings are for illustrating the specification and should not be seen as limiting the scope of the disclosure. Furthermore, this disclosure covers all possible combinations of particular or preferential embodiments herein described.

Although in the present solution only particular embodiments have been represented and described, those skilled in the art will know how to modify or change the technical characteristics to equivalent ones, depending on the requirements of each situation, without departing from the scope of protection defined by the claims below.

The embodiments herein described are combinable. The following claims set out particular embodiments of the disclosure.

## Claims

1. Composition of aqueous base for a film/solid foam for use in the treatment of urogenital infections comprising:
0.4-25 % (w/w) of a plasticizer,
0.4-20 % (w/w) of at least a mucoadhesive polymer,
at least a probiotic and/or prebiotic,
at least an active substance in pharmacologically effective amounts.

2. Composition according to the previous claim comprising:
0.5-10% (w/w) of a plasticizer,
0.5-5% (w/w) of at least a mucoadhesive polymer;
0.25 - 10% (w/w) of at least a probiotic and/or prebiotic, preferably 1-2.5% (w/w)
0.25-15 % (w/w) of an active substance, preferably 0.5-3% (w/w).

3. Composition according to the previous claims comprising 10⁵ - 10¹² CFU/ml of a probiotic culture.

4. Composition according to the previous claims wherein the probiotics are lactic acid bacteria, particularly Lactobacillus spp.

5. Composition according to the previous claim wherein the species of Lactobacillus is selected from a list consisting of: Lactobacillus iners, Lactobacillus crispatus, Lactobacillus acidophilus, Lactobacillus delbruekii, Lactobacillus jensenii or mixtures thereof.

6. Composition according to the previous claims wherein the active substance is selected from a list consisting of: metronidazole, clyndamicin, clotrimazole, chitosan, oligochitosans or combinations thereof.

7. Composition according to the previous claims further comprising a surfactant, dy, vitamin, antioxidant, hormones, and/or a cryoprotectant agent.

8. Composition according to the previous claim wherein the prebiotic is selected from a list consisting of: inulin, lactose, ascorbic acid, skim milk, fructooligosaccharides, glucose, lactic acid, sodium lactate, or combinations thereof.

9. Composition according to claims 7-8 wherein the cryoprotectant agent is selected from a list consisting of: trehalose, glycerol, lactose, glucose or combinations thereof.

10. Composition according to the previous claim wherein the mucoadhesive polymer is selected from a list consisting of: gelatin, polyvinyl alcohol, carbomers, chitosan, polycarbophils, cellulose derivatives or combinations thereof.

11. Composition according to the previous claims wherein the celulose derivatives are selected from the following list: hydroxypropylmethylcellulose, hydroxyethylcellulose, sodium carboxymethylcellulose, hydroxymethylcellulose, ethylcellulose, thyme extracts, Helichrysum spp. extracts, Echinacea spp. extracts, Satureja spp. extracts, clove extracts or combinations thereof.

12. Composition according to the previous claims wherein the chitosan has molecular weight between 10-350 kDa; preferably 10-150 kDa or 310-350 kDa.

13. Composition according to the previous claims comprising:
0.4-5% (w/w) of cellulose derivatives and,
0.1-2% (w/w) of lactic acid, or sodium lactate, or mixtures thereof.

14. Composition according to the previous claims wherein the plasticizer is a polyol, preferably selected from a list consisting of: propane-1,2 diol, propane-1,2,3 triol, glycerol, polyethyleneglycol, or combinations thereof.

15. Composition according to the previous claims for use in prevention and/or treatment of vaginal infections, particularly those caused by Gardnerella vaginalis, Prevotella spp., Mobilluncus spp., Mycoplasma hominis, Candida spp., or Trichomonas vaginalis.

16. Film obtained by dehydration of the described composition according to any one of the previous claims.

17. Film according to any one of the previous claims, with a substantially rectangular shape with length between 5-10 cm and 2-4 cm side.

18. Film according to any one of the previous claims with a thickness between 0.09 - 3 mm, preferably 0.25 - 2 mm.

19. Solid foam obtainable by lyophilization of the composition described according to any one of the previous claims.
